# EUROPEAN PATENT SPECIFICATION

(11) **EP 0 323 711 B1**
(45) Date of publication and mention of the grant of the patent: **10.03.1993**
(21) Application number: 88311417.5
(22) Date of filing: 01.12.1988
(51) Int. Cl.: A61B 5/04, A61N 1/04

(54) **Electrode with hydrogel matrix for medical use**
Medizinische Elektrode mit Hydrogelmatrix
Electrode avec matrice d'hydrogel

(30) Priority: 04.01.1988 CA 555781
(43) Date of publication of application: 12.07.1989
(73) Proprietor: LecTec Corporation, Minnetonka Minnesota (US)
(72) Inventor: Rolf, David, Minneapolis Minnesota (US)
(74) Representative: Williams, John Francis

(56) References cited:
- US-A- 4 391 278
- US-A- 4 419 998
- US-A- 4 653 503
- US-A- 4 657 023

## Description

### BACKGROUND OF THE INVENTION

United States Patents 4,274,420 and RE 31,454 describe flexible medical electrodes composed of a flexible silver foil backing to which is applied a flexible substrate or matrix containing a hydrophilic hydrocolloid, a hydrating liquid e.g. glycerin and an electrolyte to render the flexible hydrated gel electrically conductive. It has also been proposed for example in U.S. Patent Nos. 4,419,998 and 4,494,552 to provide a combination electrode capable of monitoring, defibrillation and/or stimulation medical use that includes a metal backing composed of tin having a layer of stannous chloride sprayed onto one surface. Over the stannous chloride layer is applied a porous foam disc adapted to receive an electrically conductive medium such as a saline gel. Another possibility is to locate the stannous chloride right in the conductive medium, so that it is not necessary to actually affix the stannous chloride to the conductive plate. These electrodes while generally effective have certain disadvantages. The need for a laminate of several layers of material increases production costs. Moreover, soluble substances can diffuse from one layer to another allowing chemical changes to take place during shipment, storage and use. In addition, the foam material adds to the weight and bulk of the electrode but does not assist in carrying electric current. Moreover, delamination is possible between layers which may under some circumstances interfere with the conduction of current from one layer to another. At the present time, most medical electrodes used for monitoring purposes which require application over extended periods of time include a conductive silver/silver chloride material connecting the conductive gel to the metal snap. Electrodes having tin foil backing have been used successfully in a large commercial scale only for diagnostic purposes which generally require application for only a few minutes at a time. One of the major objectives of the present invention is to find a way to construct an effective, commercially functional material electrode with a fin foil backing coated with a hydrophilic matrix containing a hydrocolloid in a hydrated state (hydrogel) that can be used both for diagnostic as well as for monitoring use over a time period of many hours and which during use will have performance characteristics not unlike the more expensive electrodes containing silver/silver chloride conductive material.

In the development work leading to the present invention, electrodes were prepared using a tin foil backing coated with a hydrophilic gel containing a tin salt such as tin chloride dissolved in the gel. Such electrodes while they were effective for some purposes did not have good shelf life for some applications. Moreover, premix solutions used in the manufacture of these gels, containing tin salts, were unstable and heterogeneous.

To understand the requirements of the present electrode, some of the problems of clinical use will be briefly reviewed. One of the desired objectives is to provide a non-silver/silver chloride containing electrode which can be used for monitoring applications. However, to be effective such an electrode must depolarize quickly after a defibrillation stimulus of approximately 200 volts is discontinued. A diagnostic electrode that remains polarized following defibrillation is ineffective in picking up EKG signals from the heart. Depolarization is particularly important since, following defibrillation with an ineffective electrode, the heart monitor trace will flip out of view on the screen and will not come back because the electrode itself looks like a battery to the monitor. Thus the monitor cannot be used to tell if a patient is recovering following defibrillation.

Another object is to provide effective coupling at the interface between the electrically conductive gel and the metal foil layer. This will permit rapid depolarization of the electrode after defibrillation. If the coupling is satisfactory, the electrode will exhibit a relatively low and constant DC offset, i.e. one that is constant over time rather than rising over a period of time after it is applied to the skin. DC offset is a minute current produced by the electrochemical makeup of the electrode itself. To be satisfactory the electrode should have a DC offset no greater than about 100 millivolts.

In addition, to be satisfactory a high performance electrode should have relatively low impedance at low frequencies, especially for EEG applications. For example, if a tin foil backing is coated with a hydrated flexible hydrophilic gel matrix containing sodium chloride as an electrolyte, the impedance will rise rapidly when the electrode is subjected to an applied alternating current as the frequency of that current decreases (say 100 to 10 cycles per second). By contrast, commercially acceptable silver/silver chloride electrodes exhibit an impedance which is substantially constant when subjected to an AC current over the same range of frequencies. An electrode will not exhibit the desired uniform impedance characteristic if its hydrophilic gel composition is incompatible or does not couple well to the metal foil backing.

The desired electrode should exhibit three effects: first, rapid depolarization of charged internal layers caused by defibrillation pulses; second, relatively low and constant DC offset and third, constant impedance with a fluctuating voltage applied at various frequencies. Low impedance is important because it will provide a lower noise to signal ratio.

### SUMMARY OF THE INVENTION

The present invention provides a medical electrode device for establishing electrical constant with the skin which includes a flexible electrically conductive backing layer composed of tin coated on its lower surface with an electrically conductive flexible substrate of a novel composition. The substrate of matrix coated on the lower surface of the tin layer is homogeneous. It is hydrophilic and sufficiently pliant to conform to the shape of the body contours when attached to the skin. The matrix includes a dispersed phase comprising about 10 percent to 50 percent of the total of the matrix formed from a hydrophilic natural or synthetic polysaccharide gum or a hydrophilic synthetic polymer or both and a liquid phase hydrating the matrix and converting the matrix to a hydrocolloidal suspension. The liquid phase includes a polyhydric alcohol and a dissolved electrolyte to render the matrix electrically conductive including at least one tin salt such as SnSO₄, SnCl₂, SnBr₂, SnBr₄, SnCl₄, SnCl₂.2H₂O and SnF₂, or the tin salts of an organic acid such as a multibasic organic acid including tin tartrate and tin citrate among others. The matrix is self supporting, i.e. it retains its shape without assistance.

A stabilizer is provided to prevent the tin salt from reacting with other chemical constituents of the matrix. While various stabilizing agents can be used, among the preferred stabilizing agents are tartaric acid and its alkali metal salts, polyacrylic acid and its alkali metal salts or other neutralized forms thereof, n-alkyl sulfonates wherein n comprises 8 to 16 carbon atoms, citric acid and its alkali metal salts, soluble nitrate salts particularly the alkali metal salts thereof and ascorbic acid and its basic salts. The stabilizing agent is preferably present in an amount at least great enough to maintain tin ions in solution within the matrix at room temperature (15°C - 25°C). These stabilizers prevent undesired tin reaction products. It was found that the tin salts dissolved in water premix solutions only initially. In a short while, precipitates form due to hydrolysis. These precipitates are amorphous tin oxides and hydroxides. This instability also lowers the pH of water premixes interfering with efficient manufacturing and consistently reliable end product. However, the stabilizers virtually eliminate these reaction products and make it possible to achieve the desired results with less of the tin salt present. This is desirable since less corrosion of the tin layer will occur, skin sensitivity will be reduced and the pH of the composition will tend to remain closer to neutral.

The invention will be described by way of example in connection with the accompanying drawings wherein:
Figure 1 is a perspective view of the upper surface of one form of electrode embodying the invention.
Figure 2 is a perspective view of the lower side of Figure 1.
Figure 3 is a perspective view of another form of electrode embodying the invention.

### DESCRIPTION OF THE PREFERRED EMBODIMENTS

Medical electrodes are used in large numbers in increasing applications such as monitoring impulses from the heart or brain. While a variety of medical electrodes of different forms and physical construction can be used in connection with the present invention, the invention will be described by way of example in two forms of snapless or tab type electrodes.

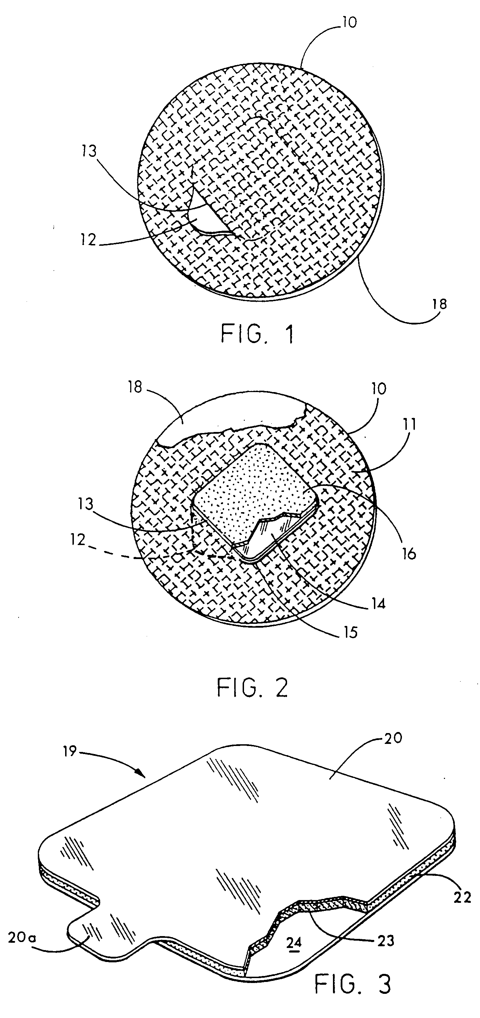

Figure 1 is a top perspective view which shows the non-adhering surface of a circular section of pressure sensitive tape 10 that has been die cut to provide a straight slit 13.

The bottom view of Figure 2 shows the relative positions of the pressure sensitive tape layer 10 and a conductive sheet 14 which comprises for example, a layer of metal or metal alloy composed of tin covered by a uniformly conductive flexible substrate or matrix 16 and a removable protective liner 18, only a small portion of which is shown so that the other components can be clearly seen. The matrix 16 is a flexible hydrated hydrophilic gel which is sufficiently pliant to conform to the shape of the body contours. The composition of the matrix will be more fully described below.

The pressure sensitive tape layer 10 is coextensive with the removable protective liner 18 and the flexible substrate or matrix 16 is coextensive with the uniformly conductive flexible tin layer 14. The tin layer 14 and the matrix 16 are located at the center of the larger pressure sensitive tape 10 such that the tape extends beyond the edge of the metallic layer 14 to provide a peripherally extending downwardly facing exposed pressure sensitive surface 11 to adhere to the skin. The pressure sensitive surface 11 at least partially surrounds the layers 14 and 16. Coextensive with the conductive layer 14 is a nonconductive backing such as a sheet 15 of plastic, e.g. polyethylene. Sheets 14 and 15 include a tab 12 which extends through, i.e. is poked through the slit 13 so as to be exposed on the upper surface of the electrode as a free appendage extending upwardly above the upper surface of the tape 10 to facilitate the connection of an electrical conductor to the electrode.

The substrate or matrix 16 is composed of a uniformly electrically conductive flexible hydrated hydrocolloidal mass capable of establishing electrical contact with the skin. It may or may not have adhesive properties.

Refer now to Figure 3 which illustrates another embodiment of the invention. In this case an electrode 19 is composed of electrically conductive layer of tin 20 in the form of flexible foil that may if desired be provided with strong plastic backing (not shown). The metallic layer 20 is provided with a laterally extending tab 20a which projects outwardly from one edge thereof and which during use extends upwardly above the upper surface of the electrode for the attachment of electrical contacts when the electrode is to be used. At least one of the surfaces of the tab 20a should be exposed so that good electrical contact can be made. Bonded to the lower surface of the tin foil layer 20 is a matrix 22 having a tacky or gummy lower surface 23, the adhesive properties of which hold the electrode in contact with the skin. Releasably bonded to the lower surface 23 of the matrix 22 is a removable cover or liner 24 which is withdrawn and discarded just before the electrode 19 is to be used. Because of its gummy or tacky lower surface 23 the matrix 22 can be thought of as a self-adhering or self-bonding matrix which adheres to the skin both through its tackiness and also through its ability to conform to the body contours and to become mechanically bonded to skin irregularities and even to extend into openings such as pores, interstices between cells and the like.

The matrix 16 or 23, as the case may be, attached to the conductive layer is substantially homogeneous. It is composed of a hydrated hydrophilic stable matrix that is sufficiently pliant to conform to the shape of the body contours. The matrix includes a dispersed phase of about 1%-50% of the total weight of the matrix formed from a hydrophilic natural or synthetic gum and/or a hydrophilic synthetic polymer. A liquid phase includes a polyhydric alcohol such as glycerin, triethylene glycol or propylene glycol or a mixture thereof. An electrolyte of any suitable salt which is non-toxic under the conditions of use is provided to render the matrix electrically conductive. The electrolyte includes at least one tin salt to be described more fully below and a stabilizer in sufficient quantity to prevent the precipitation of tin reaction products.

Of the naturally occurring gums that may be used are gum Karaya, gum acacia, locust bean gum and other polysaccharide gums and synthetically formulated gums such as modified guar gums and celluloses such as carboxymethyl cellulose, hydroxypropyl cellulose and the like. The matrix may also include synthetic polymers such as polyacrylamide, polyvinyl pyrrolodone, polyacrylic acid and modified starches such as pregelatinized starch. The synthetic polymers and/or synthetic or natural gum and other polysaccharides constitute the solid hydrated dispersed phase of the matrix.

The liquid phase of the matrix preferably comprises the aforementioned polyhydric alcohol and water. Other hydrating and solubilizing agents or humectants will be apparent to those skilled in the art. The solid portion of the matrix can comprise about 10% to about 50% of the matrix. The liquid phase composed of polyhydric alcohol and/or water comprises the balance, namely 50% to 90% of the matrix. The hydrophilic gum or hydrophilic polymer mixture thereof may be present in the amount of between 10% to 50% by weight of the matrix. All quantities and percentages stated herein are expressed as a weight percent of the total matrix. The polyhydric alcohol may be present in an amount of between 10% to 65% of the matrix and the water may be present in an amount of about 1.0% to 25% by weight. The electrolytic salt should be present in an amount sufficient to render the matrix electrically conductive. Typically, the amount of salt present is from about 1% to about 8%. The stabilizer is present in the amount of from about 0.1% to about 2% by weight.

The finished matrix is a somewhat elastic flexible hydrophilic layer that may be typically from about 0,79 mm to about 3,17 mm (about 1/32 to about 1/8 of an inch) in thickness. When applied to the skin, body moisture as well as body salts and heat are absorbed increasing its tackiness and causing the surface materials to soften. As a result the matrix of the embodiment shown in Figure 3 will flow into the pores and other irregularities in the skin creating a mechanical interlock bond with the skin in addition to the already present adhesive bond. The bonding and elastic properties of the electrode are enhanced as it ages in contact with the skin.

Several tin salts can be used. Among them are tin halides such as SnF₂, SnBr₂, SnCl₂, SnCl₄, SnBr₄ and SnCl₂.2H₂O or compounds of tin with sulfur such as SnSO₄ and organic acids including tin tartrate and tin citrate. It was found that the tin salts alone were unstable in the matrix and aqueous premixes used in manufacture. For example, a solution of SnSO₄ in water after 24 hours contains 32% insolubles which increases to about 35% in another 24 hours. If the solution is agitated the percentage of insolubles increases to 77% after 72 hours. The insolubles are believed to be primarily hydrated tin oxides and hydroxides. This is, of course, undesirable since it removed active tin ions from solution making them unavailable for electrical interactions. The formation of undesirable tin reaction products is prevented by the addition of a stabilizer in sufficient quantities to maintain the tin ions in solution at room temperature (15°C to 25°C).

Among the stabilizers that have been found suitable are tartaric acid and its alkali metal salts, polyacrylic acid and its sodium salts or other neutralized forms thereof, n-alkyl sulfonates wherein n is from 8 to 16 carbon atoms, citric acid and its alkali metal salts, soluble nitrate salts such as the alkali metal salts thereof, ascorbic acid and its basic salts, oligo or polybasic organic acids and their salts, polyethers. Stabilizers help to keep metal salts and particularly tin salts in solution by preventing undesired reaction products. Accordingly, they prevent deterioration during shipment and storage and assure that the electrical characteristic will be maintained. The stabilizers are used in an amount of 0.1% to 2%.

The polyhydric alcohol should be present in an amount between 10% to 65%. Its effect is to swell the hydrophilic polymer and help to dissolve some of the electrolytes. It is also involved in hydrogen bonding and cross linking. Too much tends to make the product mushy, runny, soft or greasy feeling, and too little causes the product to be hard and dry and usually less tacky. Propylene glycol is a less effective hydrating substance than glycerin.

Water is preferred in an amount from about 1% to 25% by weight of the matrix primarily for the purpose of swelling the hydrophilic polymers thus building viscosity and for helping to dissolve the salts to enhance their conductivity. Too much water will make the products soft, weak or over-swollen and will tend to interfere with efficient processing during the manufacturing process. If too little water is used, the matrix may lack electrical conductivity, salts may not dissolve and the product may tend to be hard and dry.

The amount of hydrophilic polysaccharide gum used can be varied from about 10% to 50% by weight. The hydrophilic polysaccharide gum build viscosity by hydrogen bonding eventually becoming semi-solid. Excessive amounts cause the formula to become too thick and viscous to be coated evenly and the product tends to be lumpy. However, if too little is used the product may be soft, mushy or runny. The gum also acts as a humectant.

The hydrophilic synthetic polymers are preferably used in an amount from about 5% to 25% of the composition to add strength and/or tackiness. One example is anionic polyacrylamide, a copolymer of acrylamide and acrylic acid. This material is sold as Reten 521PX by Hercules Chemical Company of Wilmington, Delaware. If used in excess the viscosity of the formula becomes excessive, unworkable and lumpy. If too little is used, the matrix may be soft or runny.

One suitable emulsion adhesive polymer is a copolymer of di-octylmaleate and vinyl acetate as an emulsion containing about 50% water. This product is sold under the name Flexbond 150 by the Air Products Company of Allentown, PA. These polymers if present in excess reduce pot life and cause material to become too tacky. However, if too little is used, tack may be insufficient and the product may not adhere well to the tin foil backing.

Polyacrylic acid having a molecular weight of about 50,000 is available from Poly Sciences, Inc., Warrington, PA. It assists in building viscosity by hydrogen bonding. Its low pH can be increased by adding caustic which will enhance its thickening properties. It is used in an amount of about 1% to 20% as a thickener and tackifier. It also helps in stabilizing the formulation. If too much is used, the matrix is too viscous and unworkable or lumpy. If too little is used, the composition becomes soft or runny.

Among the salt that can be used to assist in electrical conductivity, other than those already mentioned, are sodium chloride, sodium nitrate and potassium chloride. Enough should be used to render the composition a good conductor of electricity. Sodium chloride is used, for example, in amounts of from about 1% to 6% along with other salts to provide a total salt content of 7% to 8%. If too little is used, the electrode will have insufficient conductivity. If too much is used, insoluble salt particles may be present in the composition.

The tin salts already mentioned cooperate with other salts in the formulation to improve conductivity. It was also found that they assist in recovery of the electrode, i.e. depolarization following defibrillation by establishing an efficient electrical coupling at the gel-metal interface. The tin salts are usually used in an amount of from about 0.1% to 1%. If too much is used, it may be insufficiently dissolved causing cloudiness and possible skin irritation. If too little is used it may not be possible to achieve the above described performance characteristics.

The following procedure is used in compounding, extruding and curing the electrodes. It is preferred to use a premix to combine soluble salts and polar liquids such as water and glycerin. The least polar solution is first combined with the hydrophilic polymers. Progressively more polar solutions are added and mixing is continued until homogeneous. Care is taken to prevent aeration of the formula during the addition. Mixing is carried out in a pitched propeller blade agitator until homogeneous. Since the viscosity is increasing after and during mixing, it is preferred to mix the composition continuously (rather than by the batch) at a rate which material is extruded through an orifice onto the backing material for later curing. Curing is accomplished by allowing the product to remain in storage for a period of time but can be hastened by infrared radiation or other source of heat. Heating increases the rate of bond rearrangement to form a continuous network of hydrogen bonds throughout the gel.

The electrodes of the present invention provide a quick recovery following defibrillation and a relatively low level and constant impedance throughout a range of applied alternating current frequencies and exhibit DC offset stability at least equal to prior electrodes containing silver/silver chloride but have a substantially reduced cost. Moreover, since a single layer of homogeneous matrix material 16 is used production costs are no higher than the least expensive of this kind.

The invention will be better understood by reference to the following examples:

### EXAMPLE 1

| OPTIMUM WEIGHT RANGE AS A PERCENT OF THE MATRIX | | TYPICAL |
|---|---|---|
| Karaya Gum | 20-40% | 29.3% |
| Vinyl acetate based latex adhesive (Flexbond 150, Air Products, Inc., Allentown, PA) | 1-10% | 2% |
| Water | 1-10% | 8% |
| Glycerin | 40-60% | 57% |
| NaCl | 2-5% | 3% |
| SnCl₂.2H₂O | .2-1% | 0.2% |
| Sodium Tartrate Stabilizer | .2-2% | .5% |
| | | 1̅0̅0̅.̅0̅%̅ |

### EXAMPLE 2

| OPTIMUM WEIGHT RANGE AS A PERCENT OF THE MATRIX | | TYPICAL |
|---|---|---|
| Karaya Gum | 20-40% | 30% |
| Glycerin | 40-60% | 56.3% |
| Water | 1-10% | 9% |
| NaCl | 2-5% | 4% |
| SnBr₄ | .2-1% | .2% |
| n-alkyl sulfonate stabilizer | .2-2% | .5% |
| | | 1̅0̅0̅.̅0̅%̅ |

### EXAMPLE 3

| OPTIMUM WEIGHT RANGE AS A PERCENT OF THE MATRIX | | TYPICAL |
|---|---|---|
| Karaya Gum | 10-50% | 15% |
| Anionic polyacrylamide | 5-20% | 12.3% |
| Flexbond 150 | 5-20% | 9% |
| Water | 2-15% | 2% |
| NaCl | 2-8% | 2% |
| Glycerin | 50-60% | 55.0% |
| SnSO₄ | .1-1% | .2% |
| K citrate stabilizer | .5-2% | .5% |
| | | 1̅0̅0̅.̅0̅%̅ |

### EXAMPLE 4

| OPTIMUM WEIGHT RANGE AS A PERCENT OF THE MATRIX | | TYPICAL |
|---|---|---|
| Karaya Gum | 10-50% | 30% |
| Propylene glycol | 10-50% | 45% |
| Water | 10-25% | 20% |
| NaCl | 2-6% | 4% |
| SnBr₂ | .1-1% | .1% |
| Sodium nitrate stabilizer | .2-2% | .9% |
| | | 1̅0̅0̅.̅0̅%̅ |

### EXAMPLE 5

| OPTIMUM WEIGHT RANGE AS A PERCENT OF THE MATRIX | | TYPICAL |
|---|---|---|
| Anionic polyacrylamide | 10-25% | 15% |
| Vinyl acetate based latex adhesive (Flexbond 150) | 2-10% | 8.8% |
| Glycerin | 45-65% | 55% |
| Water | 7-20% | 14.5% |
| NaNO₃ | 1-5% | 4% |
| Stabilizer and electrolyte Mg(OAc)₂ | 1-4% | 2% |
| SnF₂ | .1-1% | .2% |
| NaCl | 0-6% | .5% |
| | | 1̅0̅0̅.̅0̅%̅ |

### EXAMPLE 6

| OPTIMUM WEIGHT RANGE AS A PERCENT OF THE MATRIX | | TYPICAL |
|---|---|---|
| Karaya Gum | 10-35% | 30% |
| Polyacrylic acid | 1-20% | 5% |
| Polyacrylamide | 2-10% | 5% |
| NaCl | 1-4% | 3% |
| Glycerin | 40-50% | 50% |
| SnCl₂.2H₂O | .1-1% | .5% |
| Water | 1-10% | 6% |
| Citric acid | .5-2% | .8% |
| | | 1̅0̅0̅.̅0̅%̅ |

### EXAMPLE 7

| OPTIMUM WEIGHT RANGE AS A PERCENT OF THE MATRIX | | TYPICAL |
|---|---|---|
| Polyacrylamide | 10-30% | 20% |
| Starch | 5-20% | 10% |
| Glycerin | 30-70% | 50% |
| Triethylene Glycol | 10-30% | 10% |
| NaCl | 2-6% | 3% |
| SnCl₂ | .1-1% | .2% |
| Citric acid | .5-2% | 2% |
| Water | 2-12% | 4.8% |
| | | 1̅0̅0̅.̅0̅%̅ |

### EXAMPLES 8-12

Matrices are prepared as in Example 4 except that in each successive example sodium nitrate is replaced by an equal weight of the following stabilizers: Example 8 - tartaric acid; Example 9 - sodium polyacrylate; Example 10 - citric acid; Example 11 - ascorbic acid and Example 12 - sodium ascorbate.

### EXAMPLES 13 & 14

Matrices are prepared as in Example 1 except that the sodium tartrate is replaced with the following: Example 13 - tin tartrate 0.2% by weight; Example 14 - tin citrate 0.2% by weight.

## Claims

1. A medical electrode having an upper surface and a lower surface adapted to establish electrical contact with the skin comprising,
a flexible electrically conductive tin layer (14),
said tin layer (14) including a tab (12) that is free at one end defining a portion of the edge thereof, at least the tab (12) being adapted to extend upwardly from the upper surface of the electrode (19) to facilitate the attachment of a conductor to the electrode (19),
an electrically conductive flexible substrate attached to said conductive layer (14) comprising a substantially homogeneous, hydrated hydrophilic, stable self-supporting matrix (16) sufficiently pliant to conform to the shape of the body contours,
said matrix including a dispersed phase comprising 10 percent to 50 percent of the total weight of the matrix and formed from a hydrophilic natural and/or synthetic gum and/or a hydrophilic synthetic polymer and a liquid phase hydrating the matrix and converting the matrix to a hydrocolloidal suspension,
said liquid phase comprising at least one polyhydric alcohol,
an electrolyte to render the matrix electrically conductive comprising at least one or more tin salts distributed throughout the matrix and being present at an exposed surface of the matrix adapted to contact the skin,
a stabiliser in sufficient quantity to maintain the tin ions in solution at room temperature thereby preventing the formation of insoluble tin salts, said stabiliser comprising one or more of the following: oligo or polybasic organic acids and their salts, polyethers, tartaric acid, n-alkyl sulfonate wherein n is from 8 to 16 carbon atoms, citric acid and an alkali metal salt of citric acid,
whereby said stabiliser maintains the pH of the composition, reduces corrosion of the tin layer and lessens skin sensitivity reactions.

2. The medical electrode of Claim 1 wherein the matrix (16) is surrounded by a tape layer (10) having a pressure- sensitive surface which seals the matrix (16)from the atmosphere when applied to the skin for further stabilising the matrix and said tape layer (10) helps to prevent tension applied to the tab (12) from peeling the matrix away from the skin.

3. The electrode of Claim 2 wherein the tape (10) extends across and covers the tin layer (14), said tape (10) includes an opening (13) therein and the upwardly extending tab (12) is poked through the opening (13) so as to be exposed on the upper surface of the tape (10) as a free appendage extending upwardly above the upper surface of the tape (10) to facilitate the connection of an electrical conductor thereto.

4. The electrode of claim 1,2 or 3 wherein the gum comprises karaya gum.

5. The electrode of Claim 4 wherein the karaya gum is present in an amount of from about 10% to 50% by weight of the matrix.

6. The electrode of any of claims 1 to 5, wherein the hydrophilic synthetic polymer comprises anionic polyacrylamide in an amount of from about 2% to 20% by weight of the matrix (16).

7. The electrode of any of claims 1 to 5, wherein the hydrophilic synthetic polymer comprises vinyl acetate based latex adhesive.

8. The electrode of any of claims 1 to 5, wherein the synthetic polymer is polyacrylic acid in the amount of from about 1% to about 20% by weight of the matrix (16).

9. The electrode of any of claims 1 to 5, wherein the synthetic polymer is polyvinyl pyrrolodone in the amount of from about 1% to about 15% by weight of the matrix.

10. The electrode of any preceding claim, wherein the matrix (16) comprises about 10% to 20% polyacrylamide, about 5% to 20% starch, about 40% to 70% of a polyhydric alcohol, an ionizable salt including a tin halide salt, an organic acid and water.

11. The electrode of any of the preceding Claims, wherein the tin salt is selected from:
(a) SnSO₄ SnCl₂ and SnF₄ or
(b) any one or more of SnBr₄ SnCl₄ SnBr₂ and SnCl₂.2H₂O in the amount of from about 0.1% to about 5% by weight of the matrix.

## Patentansprüche

1. Medizinische Elektrode mit einer oberen Oberfläche und einer unteren Oberfläche, geeignet zur Herstellung eines elektrischen Kontakts mit der Haut, aufweisend eine flexible elektrisch leitende Zinnschicht (14), wobei die Zinnschicht (14) eine Nase besitzt, die frei an einem Ende ist und einen Abschnitt der Kante der Zinnschicht bildet, wobei mindestens die Nase (12) geeignet ist, sich von der oberen Oberfläche der Elektrode (19) aufwärts zu erstrecken, um die Befestigung eines elektrischen Leiters an der Elektrode (19) zu erleichtern, einem elektrisch leitenden flexiblen Substrat befestigt an dem elektrischen Leiter (14), bestehend aus einer im wesentlichen homogenen, Wasser enthaltenden, hydrophilen, stabilen selbsttragenden Matrix (16), die ausreichend biegsam ist, um sich der Kontur des Körpers anzupassen, wobei die Matrix eine dispergierte Phase enthält, die 10 bis 50 Gew.% der Matrix ausmacht und gebildet wird durch einen natürlichen und/oder synthetischen hydrophilen Gummi und/oder durch ein hydrophiles synthetisches Polymer und eine flüssige Phase, die die Matrix hydratiert und die Matrix in eine hydrocolloidale Suspension umformt, wobei die flüssige Phase mindestens einen polyhidrierten Alkohol und einen die elektrische Leitfähigkeit der Matrix liefernden Elektrolyten aufweist, der mindestens ein oder mehrere in der Matrix und auf der an der Haut anliegenden Oberfläche der Matrix verteilte den Kontakt zur Haut herstellende Zinnsalze besitzt, und einen Stabilisator in genügender Menge, um die Zinnionen auch bei Raumtemperatur in Lösung zu halten und dadurch die Entstehung von ungelösten Zinnsalzen zu verhindern, wobei der Stabilisator, eine oder mehrere der folgenden Substanzen enthält: Oligo oder polybasische organische Säuren und ihre Salze, Polyäther, Weinsäure, n-Alkylsulfonate, worin n 8 bis 16 Kohlenstoffatome bedeutet, Zitronensäure und ein alkalimetallisches Salz der Zitronensäure, wobei der Stabilisator den pH-Wert der Zusammensetzung aufrechterhält, die Korrosion der Zinnschicht reduziert und Hautempfindlichkeitsreaktionen verringert.

2. Medizinische Elektrode nach Anspruch 1, in welcher die Matrix (16) von einer Tapeschicht (10) umgeben ist, ausgestattet mit einer druckempfindlichen Oberfläche, welche die Matrix (16) beim Auflegen auf die Haut von der Atmosphäre zur weiteren Stabilisierung der Matrix trennt und in welche die Tapeschicht (10) hilft, eine Belastung der Nase (12) beim Entfernen der Matrix von der Haut zu verhindern.

3. Elektrode nach Anspruch 2, in welcher das Band (10) kreuzweise gelegt ist und die Zinnschicht (14) bedeckt, das Band (10) eine Öffnung (13) enthält, durch welche die aufragende Nase (12) hindurchgesteckt wird, so daß sie freiliegend auf der oberen Oberfläche des Bandes (10) zu liegen kommt, wie ein freier Anhänger nach oben aufragend über die obere Oberfläche des Bandes (10), um die Verbindung mit einem elektrischen Leiter daran zu erleichtern.

4. Elektrode nach Anspruch 1, 2 oder 3, in welcher das Gummi Karayagummi aufweist.

5. Elektrode nach Anspruch 4, in welcher der Anteil des Karayagummis etwa 10 bis 50 Gew.% der Matrix beträgt.

6. Elektrode nach einem der Ansprüche 1 bis 5, in welcher das hydrophile synthetische Polymer ein anionisches Polyacrylamid in einer Menge von etwa 2 bis 20 Gew.% der Matrix (16) aufweist.

7. Elektrode nach einem der Ansprüche 1 bis 5, in welcher das hydrophile synthetische Polymer einen Latexkleber auf der Basis von Vinylacetat aufweist.

8. Elektrode nach einem der Ansprüche 1 bis 5, in welcher das synthetische Polymer eine Polyacrylsäure ist und etwa 1 bis 20 Gew.% der Matrix (16) ausmacht.

9. Elektrode nach einem der Ansprüche 1 bis 5, in welcher das synthetische Polymer Polyvinyl-Pyrrolidon ist und etwa 1 bis etwa 15 Gew.% der Matrix ausmacht.

10. Elektrode nach einem der vorstehenden Ansprüche, in welcher die Matrix (16) etwa 10% bis 20% Polyacrylamid, etwa 5% bis 20% Stärke, etwa 40% bis 70% eines polyhydrierten Alkohols, ein ionisierbares Zinnhalogenid enthaltendes Salz, eine organische Säure und Wasser aufweist.

11. Elektrode nach einem der vorstehenden Ansprüche, in welcher das Zinnsalz aus einem der folgenden ausgewählt ist.
(a) SnSO₄, SnCl₂ und SnF₄
oder
(b) einem oder mehreren aus SnBr₄ SnCl₄ SnBr₂ und SnCl₂.2H₂O in einer Menge von 0,1% bis etwa 5 Gew.% der Matrix.

## Revendications

1. Electrode médicale ayant une surface supérieure et une surface inférieure agencées pour établir un contact électrique avec la peau, comprenant une couche d'étain (14) souple conductrice de l'électricité, ladite couche d'étain (14) comportant une patte qui est libre à une extrémité définissant une partie du bord de celle-ci, au moins la patte (12) étant agencée pour s'étendre vers le haut à partir de la surface supérieure de l'électrode (19) pour faciliter la fixation d'un conducteur sur l'électrode (19), un substrat souple conducteur de l'électricité fixé sur la couche conductrice (14), comprenant une matrice (16) stable auto-portante, hydrophile hydratée, sensiblement homogène, suffisamment flexible pour se conformer à la forme des contours du corps, la matrice comportant une phase dispersée constituant 10 à 50 % du total en poids de la matrice et formée d'une gomme synthétique et/ou naturelle hydrophile et/ou d'un polymère synthétique hydrophile, et une phase liquide hydratant la matrice et transformant la matrice en suspension hydrocolloïdale, la phase liquide comprenant au moins un alcool polyhydrique, un électrolyte pour rendre la matrice conductrice de l'électricité et comprenant au moins un ou plusieurs sels d'étain distribués à travers la matrice et présents sur une surface exposée de la matrice agencée pour être en contact avec la peau, un stabilisateur en quantité suffisante pour maintenir les ions étain en solution à la température ambiante, empêchant ainsi la formation de sels d'étain insolubles, ledit stabilisateur comprenant un ou plusieurs des éléments suivants : les oligo-acides ou les polyacides organiques et leurs sels, les polyéthers, l'acide tartrique, le sulfonate n-alkyl dans lequel n est constitué de 8 à 16 atomes de carbone, l'acide citrique et un sel métallique alcalin de l'acide citrique, grâce à quoi le stabilisateur maintient le pH de la composition, réduit la corrosion de la couche d'étain et diminue les réactions sensibles de la peau.

2. Electrode médicale selon la revendication 1, dans laquelle la matrice (16) est entourée d'une couche de ruban (10) ayant une surface sensible à la pression, qui isole la matrice (16) de l'atmosphère lorsqu'elle est appliquée sur la peau, pour renforcer la stabilisation de la matrice, et ladite couche de ruban (10) aide à empêcher qu'une traction appliquée sur la patte (12) ne détache la matrice de la peau.

3. Electrode selon la revendication 2, dans laquelle le ruban (10) s'étend sur la couche d'étain (14) et la recouvre, ledit ruban (10) comportant une ouverture (13), la patte (12) s'étendant vers le haut en étant insérée à travers l'ouverture (13) de manière à être exposée sur la surface supérieure du ruban (10) en formant un élément libre s'étendant vers le haut au dessus de la surface supérieure du ruban (10) pour faciliter la connexion d'un conducteur électrique.

4. Electrode selon la revendication 1, 2 ou 3 dans laquelle la gomme est de la gomme karaya.

5. Electrode selon la revendication 4, dans laquelle la gomme karaya est présente dans une quantité d'environ 10 à 50 % en poids de la matrice.

6. Electrode selon l'une quelconque des revendications 1 à 5, dans laquelle le polymère synthétique hydrophile comprend un polyacrylamide anionique dans une quantité d'environ 2 à 20 % en poids de la matrice (16).

7. Electrode selon l'une des revendications 1 à 5, dans laquelle le polymère synthétique hydrophile est un adhésif de latex à base d'acétate de vinyle.

8. Electrode selon l'une quelconque des revendications 1 à 5, dans laquelle le polymère synthétique est de l'acide polyacrylique en quantité d'environ 1 à 20 % en poids de la matrice (16).

9. Electrode selon l'une quelconque des revendications 1 à 5, dans laquelle le polymère synthétique est du polyvinyl pyrrolidone en quantité d'environ 1 à 15 % en poids de la matrice.

10. Electrode selon l'une quelconque des revendications précédentes, dans laquelle la matrice (16) comprend environ 10 à 20 % de polyacrylamide, environ 5 à 20 % d'amidon, environ 40 à 70 % d'alcool polyhydrique, un sel ionisable comprenant un sel d'halogénure d'étain, un acide organique et de l'eau.

11. Electrode selon l'une quelconque des revendications précédentes, dans laquelle le sel d'étain est choisi dans le groupe constitué de :
(a) SnSO₄, SnCl₂, et SnF₄ ou
(b) de l'un ou plusieurs des sels suivants : SnBr₄, SnCl₄, SnBr₂ et SnCl₂,2H₂O en quantité d'environ 0,1 à 5 % en poids de la matrice.
